# EUROPEAN PATENT APPLICATION

(11) **EP 4 759 302 A1**
(43) Date of publication of application: **17.06.2026**
(21) Application number: 24851030.7
(22) Date of filing: 07.08.2024
(51) Int. Cl.: A61K 9/107, A61K 39/39

(54) **IMMUNE-ENHANCING ALUMINUM EMULSION, AND PREPARATION METHOD THEREFOR AND USE THEREOF**

(30) Priority: 07.08.2023 CN 202310984025
(71) Applicant: Xiamen University, Xiamen, Fujian 361005 (CN); Beijing Wantai Biological Pharmacy Enterprise Co., Ltd., Beijing 102206 (CN); Xiamen Innovax Biotech Co., Ltd., Xiamen, Fujian 361022 (CN)
(72) Inventor: ZHANG, Tianying, Xiamen, Fujian 361102 (CN); NIE, Meifeng, Xiamen, Fujian 361102 (CN); WU, Shuyu, Xiamen, Fujian 361102 (CN); WU, Junwei, Xiamen, Fujian 361102 (CN); CHEN, Ruitong, Xiamen, Fujian 361102 (CN); YUAN, Quan, Xiamen, Fujian 361102 (CN); LI, Shaowei, Xiamen, Fujian 361102 (CN); ZHANG, Jun, Xiamen, Fujian 361102 (CN); XIA, Ningshao, Xiamen, Fujian 361102 (CN)
(74) Representative: Meissner Bolte Partnerschaft mbB
(86) International application number: PCT/CN2024/110337
(87) International publication number: WO 2025/031385

(57) **Abstract**

An immune-enhancing aluminum emulsion, and a preparation method therefor, and a use thereof. Specifically provided are an oil-in-water emulsion containing an inorganic salt and a preparation method for the oil-in-water emulsion. Also provided are a vaccine adjuvant comprising the oil-in-water emulsion, a vaccine composition, and a pharmaceutical composition. Also provided is a use of the oil-in-water emulsion as a vaccine adjuvant.

## Description

### Cross-Reference to Related Application

The present application is based on and claims priority to the Chinese application with application number CN 202310984025.9 (filed on August 7, 2023). The disclosure of the Chinese application is incorporated into the present application in its entirety.

### Technical Field

The present invention belongs to the field of pharmaceutical technology. Specifically, the present invention relates to an immune-enhancing aluminum emulsion, and preparation method therefor and use thereof.

### Background Art

Adjuvants are substances that can specifically or non-specifically bind to immunogens, inducing a long-term, effective, specific immune response in the body, thus playing an auxiliary role. The immunobiological effects of adjuvants include reducing the amount of immunogen used, enhancing the immunogenicity of antigens, and altering the type of immune response. Common types of adjuvants include mineral salt adjuvants such as aluminum salts, emulsion adjuvants, liposomal adjuvants, and virosomal adjuvants.

Aluminum salt adjuvants are recognized as the most widely used, safe, and effective adjuvants. Aluminum salts mainly include aluminum phosphate, aluminum hydroxide, and potassium aluminum sulfate. Currently, the commonly used aluminum adjuvants are aluminum hydroxide and aluminum phosphate. Aluminum adjuvants are widely used in human vaccines. However, aluminum adjuvants also have drawbacks. For example, while they are generally effective in increasing serum antibodies, they can cause injection site reactions, and their ability to induce cellular immunity is relatively weak, which limits their application to some extent. Furthermore, for certain vaccines (e.g., influenza), aluminum salt adjuvants are not significantly effective.

Besides aluminum salt adjuvants, another type of commercially available adjuvant widely used in clinical practice is emulsion-type adjuvants (emulsion adjuvants). Emulsion adjuvants are emulsions formed by mixing oils and surfactants in a certain proportion, mainly composed of oils, surfactants, and water. Commonly available emulsion adjuvants on the market include Freund's adjuvant, white oil-Span adjuvant, MF-59, ISA 206, ISA720, Adjuvant-65, SAF, etc., among which oil-in-water adjuvants are often used in human vaccines. Emulsion adjuvants can promote the production of high-titer antibodies against a variety of antigens, relatively prolong the duration of continuous antigen stimulation, reduce the dosage of antigen for vaccination, and decrease the frequency of antigen administration. Due to these advantages, emulsion adjuvants are widely used in animal vaccines. However, while these adjuvants function effectively in vivo, they also present some safety risks, such as tissue damage, stress responses, mineral oil residues, and potential carcinogenicity.

With the continuous emergence of new vaccines, the requirements for vaccine adjuvants are becoming increasingly stringent. The development of vaccine adjuvants needs to balance safety and efficacy while also meeting the demands of large-scale production.

### Contents of the Invention

Due to the heat sensitivity of squalene, adjuvants containing squalene cannot be sterilized by hydrothermal sterilization, and filtration sterilization is the best option. Existing emulsions prepared from inorganic salts and emulsifiers have particle sizes in the micrometer range or larger than 220 nm, making it impossible to use filtration processes for sterilization. The present invention provides an inorganic salt particle-encapsulating emulsion with a particle diameter of less than 220 nm, and thus it can be sterilized through filtration, ensuring the sterility of adjuvant. Furthermore, this novel, filterable, nanoscale adjuvant exhibits a stronger immune-enhancing effect than traditional aluminum adjuvants. The present invention is thus provided as follows:
In one aspect, the present application provides an inorganic salt-containing oil-in-water emulsion, wherein the inorganic salt is one or more selected from the group consisting of aluminum salt, zinc salt, calcium salt, magnesium salt, iron salt, and manganese salt; the emulsion comprises an aqueous phase which is selected from the group consisting of acetic acid-sodium acetate buffer solution, physiological saline, Tris-HCl buffer solution, HEPES-HCl buffer solution, and histidine-HCl buffer solution; the emulsion comprises an oil phase which is one or more selected from the group consisting of squalene and squalane; the emulsion further comprises a surfactant, and the oil phase/surfactant mass ratio is less than 5.1; the inorganic salt and surfactant are distributed at the interface between the oil phase and the aqueous phase; the effective particle size of the inorganic salt-containing emulsion is less than 220 nm.

In the emulsion of the present invention, squalene and/or squalane is used as the oil phase. Squalene is a triterpenoid compound with an isoprene-type molecular structure having 30 carbon atoms and 50 hydrogen atoms, its systematically name is 2,6,10,15,19,23-hexamethyl-2,6,10,14,18,22-tetracosahexaene, and its molecular formula is C₃₀H₅₀. Squalene can be derived from animal or plant extracts or chemically synthesized. Squalene is a metabolizable oil, and emulsion adjuvants containing squalene, such as MF59 and AS03, exhibit excellent immune-enhancing effects. Squalane, a hydrocarbon oil obtained by hydrogenating squalene, has the molecular formula C₃₀H₆₂ and can also be used as the oil phase of emulsion adjuvant. In some embodiments, the oil phase of the emulsion of the present invention is squalene.

The inorganic salt used in the present invention can act as an immunoadjuvant, producing a synergistic immune-enhancing effect with the oil-in-water emulsion, reducing antigen dosage, increasing antibody level, and increasing antibody type diversity as well. On the other hand, the inorganic salt can stabilize the emulsion, contributing to the formation of smaller and more stable droplets. The inorganic salt used in the present invention can be an inorganic metal salt, such as one or more selected from the group consisting of aluminum salt, zinc salt, calcium salt, magnesium salt, iron salt, and manganese salt, and can be a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate. In some embodiments, the inorganic salt is aluminum chloride.

The aqueous phase of the emulsion of the present invention can be physiological saline or a buffer solution, such as acetic acid-sodium acetate buffer solution, Tris-HCl buffer solution, HEPES-HCl buffer solution, or histidine-HCl buffer solution. Using physiological saline or the above buffer solutions is beneficial for forming smaller and more uniform droplets. Since squalene is heat-sensitive, adjuvants containing squalene cannot be sterilized by hydrothermal sterilization; and filtration sterilization is a better choice. A filter with a 220 nm pore size can be used for sterilization if the emulsion particle size is controlled below 220 nm.

In some embodiments, the buffer solution has a concentration of 20 to 100 mM (e.g., 20 to 30 mM, 30 to 40 mM, 40 to 50 mM, 50 to 60 mM, 60 to 70 mM, 70 to 80 mM, 80 to 90 mM, or 90 to 100 mM).

The emulsion of the present invention further comprises a surfactant to stabilize the emulsion and prevent demulsification. Commonly used surfactants in the art can be selected, such as polyoxyethylene sorbitan ester surfactant (commonly known as Tween), sorbitan ester (commonly known as Span), octoxynol-99 (Triton X-100 or tert-octylphenoxypolyethoxyethanol), lecithin, etc. In some embodiments, the surfactant is selected from the group consisting of Tween 80 (polyoxyethylene sorbitan monooleate), Span 85 (sorbitan trioleate), or combination thereof.

The inventors have discovered that the ratio of the oil phase to the surfactant affects the particle size of the emulsion of the present invention. An oil phase/surfactant mass ratio of less than 5.1 makes it easier to obtain emulsions with a particle size below 220 nm. In some embodiments, the oil phase/surfactant mass ratio is 1.6 to 2.5, 2.5 to 2.6, 2.6 to 3.3, 3.3 to 3.4, 3.4 to 4.1, 4.1 to 4.3, or 4.3 to 5.0. In some embodiments, the oil phase/surfactant mass ratio is less than 4.1.

In the present invention, the particle size of the emulsion refers to the average particle size of the droplets contained in the emulsion. The particle size of the emulsion in the present invention can be expressed as D10, D50, D90, or effective particle size (Eff. Diam.).

In some embodiments, the particle size of the emulsion of the present invention is expressed as D10, which is 90 to 130 nm (e.g., 90 to 100 nm, 100 to 110 nm, 110 to 120 nm, or 120 to 130 nm).

In some embodiments, the particle size of the emulsion of the present invention is expressed as D50, which is 170 to 200 nm (e.g., 170 to 180 nm, 180 to 190 nm, or 190 to 200 nm).

In some embodiments, the particle size of the emulsion of the present invention is expressed as effective particle size. In the present invention, the effective particle size is defined as effective diameter, which is calculated based on the experimentally determined translational diffusion coefficient using the Stokes-Einstein equation. The translational diffusion coefficient is obtained by calculating the cumulative autocorrelation function of the scattered light using the cumulant method. The effective particle size can be obtained using a light scattering particle size analyzer (e.g., Omni multi-angle particle size and high-sensitivity Zeta potential analyzer manufactured by NanoBrook). In some embodiments, the effective particle size of the emulsion of the present invention is 50 to 60 nm, 60 to 70 nm, 70 to 80 nm, 80 to 90 nm, 90 to 100 nm, 100 to 110 nm, 110 to 120 nm, 100 to 110 nm, 150 to 200 nm, or 200 to 220 nm.

The emulsion of the present invention is an oil-in-water emulsion, wherein the oil is the internal phase dispersed in the aqueous phase, the aqueous phase is the continuous external phase, and the inorganic metal salt and surfactant are distributed at the interface between the oil phase and the aqueous phase. Under a transmission electron microscope, the emulsion droplets appear as spherical particles with metal salt particles distributed on the surface and outer side.

The emulsion of the present invention exhibits good particle size uniformity. In some embodiments, the PDI (polydispersity index) of the emulsion of the present invention is less than 0.2 (e.g., 0.01 to 0.05, 0.05 to 0.1, 0.1 to 0.15, or 0.15 to 0.2).

The inventors discovered that by controlling the content of inorganic metal element to less than 1200 ppm, it is easier to obtain a metal salt-encapsulating emulsion with a particle size of less than 220 nm. In some embodiments, the content of inorganic metal element in the emulsion of the present invention is 50 to 1200 ppm (e.g., 50 to 60 ppm, 60 to 70 ppm, 70 to 80 ppm, 80 to 90 ppm, 90 to 95 ppm, 95 to 98 ppm, 98 to 100 ppm, 100 to 103 ppm, 103 to 105 ppm, 105 to 110 ppm, 110 to 150 ppm, 150 to 200 ppm, 200 to 300 ppm, 300 to 400 ppm, 400 to 500 ppm, 500 to 600 ppm, 600 to 700 ppm, 700 to 800 ppm, 800 to 900 ppm, 900 to 1000 ppm, 1000 to 1100 ppm, or 1100 to 1200 ppm).

The inventors discovered that when the surfactant in the emulsion contains Tween 80, adjusting the concentration of Tween 80 to greater than 0.3% (w/v) makes it easier to obtain a metal salt-encapsulating emulsion with a particle size of less than 220 nm. In some embodiments, the concentration of Tween 80 in the emulsion of the present invention is 0.3% (w/v) to 0.35% (w/v), 0.35% (w/v) to 0.4% (w/v), 0.4% (w/v) to 0.45% (w/v), 0.45% (w/v) to 0.5% (w/v), 0.5% (w/v) to 0.55% (w/v), or 0.55% (w/v) to 0.6% (w/v).

In some embodiments, the emulsion of the present invention further comprises an additional immunostimulant or peptide, such as CpG1018, MPL, PolyIC, cGAMP, etc., to further enhance the immune response. In some embodiments, the additional immunostimulant or peptide is added at a concentration of 2.5 to 10 µg/mL (e.g., 2.5 to 5 µg/mL, 5 to 7.5 µg/mL, or 7.5 to 10 µg/mL).

The oil-in-water emulsion of the present invention exhibits good stability. In some embodiments, after storage at 4 °C for at least 4 months, the particle size change of the emulsion does not exceed 10 nm, and it does not separate into layers.

In one aspect, the present application also relates to two methods for preparing an inorganic salt-containing oil-in-water emulsion. The first method comprises preparing an emulsion and an inorganic metal salt solution separately, and then mixing them to form the inorganic metal salt-containing emulsion. The second method comprises incorporating an inorganic metal salt into the formation of either an oil phase or an aqueous phase to prepare the inorganic metal salt-containing emulsion via a one-step process.

Therefore, the present application relates to a method for preparing an inorganic salt-containing oil-in-water emulsion, the method comprising the following steps:
S1: preparing an oil-in-water emulsion using acetic acid-sodium acetate buffer solution, physiological saline, Tris-HCl buffer solution, HEPES-HCl buffer solution, or histidine-HCl buffer solution as the aqueous phase, squalene and/or squalane as the oil phase, and a surfactant; wherein the oil phase/surfactant mass ratio is less than 5.1; the emulsion has an effective particle size of less than 220 nm, and a PDI of 0.07 to 0.2 (e.g., 0.07~0.08, 0.08~0.09, 0.09~0.1, 0.1~0.12, 0.12~0.14, 0.14~0.16, 0.16~0.18, or 0.18~0.2);
S2: adding an inorganic salt solution dropwise to the emulsion prepared in S1; wherein the inorganic salt is one or more selected from the group consisting of aluminum salt, zinc salt, calcium salt, magnesium salt, iron salt, and manganese salt; the solvent of the inorganic salt solution is the same as the aqueous phase in S1; the concentration of the inorganic salt in the inorganic salt solution is less than 450 mM; after the addition is complete, stirring is continued (e.g., stirring is carried out for 10 min), and followed by sterile filtration using a 0.22 µm filter.

In some embodiments, the inorganic salt is a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate.

In S1, the surfactant can be a commonly used one in the art, such as Tween, Span, Triton X-100, and lecithin. In some embodiments, the surfactant is selected from the group consisting of Tween 80, Span 85, or combination thereof.

The mixing of the oil phase and the aqueous phase can be performed using a method such as microfluidic emulsification, homogenization, ultrasonic emulsification, dual-syringe push emulsification, spray emulsification, microfluidization, microchannel emulsification, membrane emulsification, stirring, shaking, inversion or hand-shaking mixing. In some embodiments, the oil phase and the aqueous phase are mixed by high-speed shearing.

In some embodiments, the concentration of the buffer solution used in S1 is 20 to 100 mM (e.g., 20 to 30 mM, 30 to 40 mM, 40 to 50 mM, 50 to 60 mM, 60 to 70 mM, 70 to 80 mM, 80 to 90 mM, or 90 to 100 mM).

In some embodiments, S1 comprises: suspending surfactant 1 (e.g., Tween 80) in a certain volume of buffer solution (e.g., acetic acid-sodium acetate buffer solution) or physiological saline, and defining it as solution A; suspending surfactant 2 (e.g., Span 85) in squalene and/or squalane, and defining it as solution B; adding solution A to solution B, performing high-speed shearing to form a crude emulsion, and then performing homogenization for several times (e.g., three times) to form emulsion 1.

The surfactant 1 and surfactant 2 may be the same or different, and may be selected from the group consisting of the surfactants described above.

In some embodiments, the content of surfactant 1 (e.g., Tween 80) in emulsion 1 is 0.1% to 0.5% (w/v) (e.g., 0.1% to 0.2%, 0.2% to 0.3%, 0.3% to 0.4%, or 0.4% to 0.5%).

In some embodiments, the content of surfactant 2 (e.g., Span 85) in emulsion 1 is 0.1% to 0.5% (w/v) (e.g., 0.1% to 0.2%, 0.2% to 0.3%, 0.3% to 0.4%, or 0.4% to 0.5%).

In some embodiments, the volume content of squalene and/or squalane in emulsion 1 is 1% to 5% (v/v) (e.g., 1% to 2%, 2% to 3%, 3% to 4%, or 4% to 5%).

In some embodiments, shearing is performed using a high-pressure homogenizer. In some embodiments, shearing is performed at 8000 to 12000 rpm (e.g., 10000 rpm) for 2 to 5 minutes.

In some embodiments, homogenization is performed using a high-pressure microfluidic nano-homogenizer.

In some embodiments, the concentration of the buffer solution (e.g., acetic acid-sodium acetate buffer solution) is 20 to 100 mM (e.g., 20 to 30 mM, 30 to 40 mM, 40 to 50 mM, 50 to 60 mM, 60 to 70 mM, 70 to 80 mM, 80 to 90 mM, or 90 to 100 mM).

In S2, when the concentration of the inorganic metal salt solution is less than 450 mM, it is more conducive to obtaining an emulsion with a particle size less than 220 nm. In some embodiments, the concentration of the inorganic metal salt solution is less than 18.75 mM, 18.75 mM to 37.5 mM, 37.5 mM to 75 mM, 75 mM to 112.5 mM, 112.5 mM to 225 mM, 225 mM to 337.5 mM, or 337.5 mM to 450 mM. In some embodiments, S2 comprises: dissolving the inorganic metal salt in a buffer solution (e.g., acetic acid-sodium acetate buffer solution) or physiological saline to obtain the inorganic metal salt solution; adding the inorganic metal salt solution dropwise at a volume ratio of 1:9 to 1:10 at a rate of 100 to 500 µL/time to the emulsion obtained in step (1), continuously stirring after the addition is complete, and then sterilizing by filtration through a 0.22 µm filter.

In some embodiments, the concentration of the inorganic metal salt in the inorganic metal salt solution is 18.75 mM to 450 mM (e.g., 18.75 mM to 37.5 mM, 37.5 to 50 mM, 50 to 100 mM, 100 to 200 mM, 200 to 300 mM, 300 to 400 mM, or 400 to 450 mM).

In some embodiments, the concentration of the buffer solution (e.g., acetic acid-sodium acetate buffer solution) used in S2 is 20 to 100 mM (e.g., 20 to 30 mM, 30 to 40 mM, 40 to 50 mM, 50 to 60 mM, 60 to 70 mM, 70 to 80 mM, 80 to 90 mM, or 90 to 100 mM).

In some embodiments, in S2, the volume ratio of the inorganic metal salt solution to the emulsion used and obtained in S1 is 1:9, 1:9.1, 1:9.2, 1:9.3, 1:9.4, 1:9.5, 1:9.6, 1:9.7, 1:9.8, 1:9.9, or 1:10.

In some embodiments, the inorganic metal salt solution is added dropwise to the emulsion obtained in step (1) at a rate of 100 to 200 µL/time, 200 to 300 µL/time, 300 to 400 µL/time, or 400 to 500 µL/time.

In some embodiments, during the dropwise addition, the emulsion obtained in step (1) is stirred at a speed of 500 to 1000 rpm (e.g., 500 rpm, 600 rpm, 700 rpm, 800 rpm, 900 rpm, or 1000 rpm).

In some embodiments, stirring continues for 10 to 60 minutes after the dropwise addition.

In some embodiments, after filtration, refrigeration (e.g., at 4 °C) is performed for storage and later use.

The present application relates to another method for preparing an inorganic salt-containing oil-in-water emulsion, the method comprising the following steps:
S1': suspending a first surfactant and an inorganic salt in an acetic acid-sodium acetate buffer solution, physiological saline, Tris-HCl buffer solution, HEPES-HCl buffer solution, or histidine-HCl buffer solution, and defining it as aqueous phase; mixing a second surfactant with squalene and/or squalane, and defining it as oil phase; or,
suspending a first surfactant in an acetic acid-sodium acetate buffer solution, physiological saline, Tris-HCl buffer solution, HEPES-HCl buffer solution, or histidine-HCl buffer solution, and defining it as aqueous phase; mixing a second surfactant, as well as squalene and/or squalane with an inorganic salt, and defining it as oil phase;
S2': mixing the aqueous phase and the oil phase, performing high-speed shearing to form a crude emulsion, then performing homogenization for several times to form an inorganic salt-encapsulating emulsion, and performing sterile filtration using a 0.22 µm filter.

In some embodiments, the inorganic salt in S1' is a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate.

In S1', the surfactant is a commonly used one in the art, such as Tween, Span, Triton X-100, lecithin. In some embodiments, the surfactant is selected from the group consisting of Tween 80, Span 85, or combination thereof. In some embodiments, the first surfactant and the second surfactant are Tween 80 and Span 85, respectively.

The mixing of the oil phase and the aqueous phase can be carried out using a method such as microfluidic emulsification, homogenization, ultrasonic emulsification, dual-syringe push emulsification, spray emulsification, microfluidization, microchannel emulsification, membrane emulsification, stirring, shaking, inversion or hand-shaking mixing. In some embodiments, the oil phase and the aqueous phase are mixed by high-speed shearing.

In S1', the concentrations of the inorganic salt and the surfactant in the aqueous phase or oil phase can be adjusted to obtain an emulsion with a smaller particle size.

In some embodiments, the inorganic salt content in the aqueous phase is 1.92 to 46.2 mM, for example 1.92 to 3 mM, 3 to 7 mM, 7 to 10 mM, 10 to 15 mM, 15 to 20 mM, 20 to 25 mM, 25 to 30 mM, 30 to 35 mM, 35 to 40 mM, or 40 to 46.2 mM.

In some embodiments, the inorganic salt content in the oil phase is 35.2 to 845 mM, for example 35.2 to 50 mM, 50 to 100 mM, 100 to 150 mM, 150 to 200 mM, 200 to 250 mM, 250 to 300 mM, 300 to 350 mM, 350 to 400 mM, 400 to 450 mM, 450 to 500 mM, 500 to 550 mM, 550 to 600 mM, 600 to 650 mM, 650 to 700 mM, 700 to 750 mM, 750 to 800 mM, or 800 to 845 mM.

In some embodiments, the first surfactant is present in the inorganic salt-encapsulating emulsion at a concentration of 0.1% to 0.5% (w/v), for example, 0.1% to 0.2%, 0.2% to 0.3%, 0.3% to 0.4%, or 0.4% to 0.5%.

In some embodiments, the second surfactant is present in the inorganic salt-encapsulating emulsion at a concentration of 0.1% to 0.5% (w/v), for example, 0.1% to 0.2%, 0.2% to 0.3%, 0.3% to 0.4%, or 0.4% to 0.5%.

In some embodiments, a benchtop homogenizer is used for shearing. In some embodiments, shearing is performed at 8000 to 12000 rpm (e.g., 10000 rpm) for 2 to 5 minutes.

In some embodiments, homogenization is performed several times (e.g., three times) using a high-pressure microfluidic nano-homogenizer.

Through the above method of the present invention, an inorganic salt-containing oil-in-water emulsion with a particle size of less than 220 nm can be obtained.

In one aspect, the present application also relates to an immunogenic composition, which comprises the inorganic salt-containing oil-in-water emulsion of the present invention and one or more antigens.

The immunogenic composition as used herein, when administered to a subject or animal, is capable of inducing a protective immune response against the one or more antigens contained therein.

In one aspect, the present application also relates to a vaccine composition, which comprises the inorganic salt-containing oil-in-water emulsion of the present invention and one or more antigens.

The vaccine composition as used herein, when administered to a subject or animal, is capable of inducing a protective immune response against, for example, a microorganism, or is capable of effectively protecting the subject or animal from infection.

The vaccine composition may be a prophylactic or therapeutic vaccine. Preferably, the vaccine composition comprises a genetically engineered vaccine, such as protein vaccine, such as respiratory coronavirus S protein, varicella-zoster virus gE antigen.

In one aspect, the present application also relates to a vaccine adjuvant, which comprises the inorganic salt-containing oil-in-water emulsion of the present invention. For example, such vaccine adjuvant may also comprise a secondary adjuvant as described below.

As used herein, the term "adjuvant" or "vaccine adjuvant" is intended to refer to a substance that can nonspecifically accelerate, prolong, or enhance an immune response to an antigen.

Advantageously, the adjuvant can also reduce the number of immunizations or the amount of antigen required for protective immunization.

It is well known that adjuvants themselves do not or hardly induce an immune response, but adjuvants can enhance an immune response to an antigen. Therefore, the inorganic salt-containing oil-in-water emulsion of the present invention can be combined with one or more antigens to produce an immunogenic composition or vaccine that can be used to induce an immune response in an individual. A variety of substances, for example, attenuated and inactivated viral and bacterial pathogens, purified macromolecules, proteins, polysaccharides, toxoids, recombinant antigens, organisms containing foreign genes from pathogens, synthetic peptides, polynucleotides, antibodies, and tumor cells, can be used as antigens in the immunogenic or vaccine-type composition or formulation. Antigens can be used in both prophylactic and therapeutic vaccines. Antigens include protein antigens, such as respiratory coronavirus S protein, varicella-zoster virus gE glycoprotein antigen, and respiratory syncytial virus protein (RSV).

Various immunomodulatory molecules can also be used in combination with the inorganic salt-containing oil-in-water emulsion of the present invention to alter the immune response in an individual. The immunomodulators described herein refer to a class of preparations capable of regulating, balancing, and restoring the immune function in the body. Commonly used immunomodulators fall into three main categories: immunostimulants, immunosuppressants, and bidirectional immunomodulators.

The amounts and dosages of the antigen and the inorganic salt-containing oil-in-water emulsion in the vaccine composition of the present invention are determined using techniques known to those skilled in the pharmaceutical art, taking into account factors such as the specific antigen, the age, sex, weight, species, and condition of the specific animal or patient, and the route of administration.

In a preferred embodiment, the vaccine composition of the present invention further comprises one or more components selected from the group consisting of: surfactant, absorption enhancer, water-absorbing polymer, substance capable of inhibiting enzymatic degradation, alcohol, organic solvent, oil, pH control agent, preservative, osmotic pressure control agent, propellant, water, and any mixture thereof.

The vaccine composition of the present invention may also comprise a pharmaceutically acceptable carrier. The amount of the carrier will depend on the selected amounts of other components, the required concentration of antigen, the choice of route of administration (oral or parenteral), etc. The carrier can be added to the vaccine at any convenient time. In the case of lyophilized vaccines, the carrier can be added, for example, just before administration. Alternatively, the final product can be manufactured with the carrier.

Examples of suitable carriers include, but are not limited to, sterile water, saline, buffer solution, phosphate-buffered saline, buffered sodium chloride, vegetable oil, minimum essential culture medium (MEM), HEPES-containing MEM, etc.

Optionally, the vaccine composition of the present invention may contain varying amounts of conventional secondary adjuvants, depending on the adjuvant and the desired outcome.

Examples of suitable secondary adjuvants include, but are not limited to, stabilizer; emulsifier; pH adjuster such as sodium hydroxide, hydrochloric acid, etc.; surfactant such as Tween.RTM. 80 (polysorbate 80, commercially available from Sigma Chemical Co. (St. Louis, Mo.)); liposome; iscom adjuvant; synthetic glycopeptide such as muramyl dipeptide; extender such as dextran; carboxyvinyl polymer; bacterial cell wall such as mycobacterial cell wall extract; their derivatives such as *Corynebacterium parvum; Propionibacterium acne; Mycobacterium bovis,* for example, *Bovine Calmette Guerin* (BCG); vaccinia or animal poxvirus protein; subviral particle adjuvant such as circovirus; cholera toxin; N,N-dioctadecyl-N',N'-bis(2-hydroxyethyl)-propanediamine (pyridine); monophospholipid A; dimethyldioctadecylammonium bromide (DDA, commercially available from Kodak (Rochester, N.Y.)); and their compositions and mixtures.

Examples of suitable stabilizers include, but are not limited to, sucrose, gelatin, peptone, and digested protein extracts such as NZ-amine or NZ-amine AS. Examples of emulsifiers include, but are not limited to, mineral oil, vegetable oil, peanut oil, and other standard, metabolizable, non-toxic oils that can be used in injectable or intranasal vaccine compositions.

For the purposes of the present invention, these adjuvants are referred to herein as "secondary" only in contrast to the above-described inorganic salt-containing oil-in-water emulsion, because the inorganic salt-containing oil-in-water emulsion, when combined with an antigen, can significantly enhance the immune response against that antigen, thus making it an essential component of the vaccine composition. Secondary adjuvants are primarily included in vaccine formulations as processing aids, although some adjuvants do possess some degree of immunomodulatory properties, thus serving a dual purpose.

Conventional preservatives can be added to the vaccine composition in an effective amount of about 0.0001% (by weight) to about 0.1% (by weight). Depending on the preservative used in the formulation, amounts below or above this range may also be used. Typical preservatives include, for example, potassium sorbate, sodium pyrosulfite, phenol, methylparaben, propylparaben, thimerosal, etc.

The selection of inactivated, modified, or other types of vaccine compositions, and the methods for preparing the improved vaccine composition formulations of the present invention, are known or readily determined by those skilled in the art.

Generally, the vaccine compositions of the present invention are conveniently administered via oral, parenteral (subcutaneous, intramuscular, intravenous, intradermal, intraperitoneal, or intra-peritoneal), oral, intranasal, or transdermal routes. The administration routes contemplated by the present invention will depend on the antigen and adjuvant. For example, if the vaccine composition contains saponins, although they are non-toxic when administered orally or intranasally, care must be taken to avoid injecting saponin glycosides into the bloodstream, as they act as strong hemolytic agents. Furthermore, many antigens will not be effective if administered orally. Preferably, the vaccine composition is administered via the intramuscular route.

The dosage of the vaccine composition will depend significantly on the chosen antigen, route of administration, species, and other standard factors. It is envisioned that one of ordinary skill in the art can readily titrate the appropriate dosage for the immune response against each antigen to determine the effective immunizing amount and method of administration.

As a vaccine adjuvant, the inorganic salt-containing oil-in-water emulsion of the present invention can improve vaccine efficacy by enhancing the immunogenicity of weaker antigens (e.g., highly purified or recombinant antigens), reducing the amount of antigen required for immune response, and reducing the frequency of immunization required to provide protective immunity, and can also improve vaccine efficacy in individuals with reduced or weakened immune responses (e..g, newborns, the elderly, and immunocompromised individuals), enhance immunogenicity in target tissues, or promote cell-mediated immunity and/or humoral immunity by triggering a specific cytokine profile.

Combinations of antigens and/or immunomodulatory molecules with the inorganic salt-containing oil-in-water emulsion of the present invention can be tested in a variety of preclinical toxicology and safety studies known in the art.

For example, such combinations can be evaluated in animal models in which the antigen has been found to be immunogenic and which animal models can be reproducibly immunized using the same approach proposed for human clinical trials.

Combinations of antigens and/or immunomodulatory molecules with the inorganic salt-containing oil-in-water emulsion of the present invention can be tested, for example, using protocols proposed by the Center for Biological Evaluation and Research of the U.S. Food and Drug Administration and the National Institute of Allergy and Infectious Diseases (Goldenthal, KL et al. AID Res Hum Retroviruses, 9:S45-9 (1993)).

Those skilled in the art will know how to determine appropriate antigen load, route of immunization, dosage, antigen purity, and vaccination regimen that can be used to treat a specific pathological condition in a specific animal species for a particular combination of antigen and/or immunomodulatory molecule with the compound adjuvant of the present invention.

The immunogenic composition or vaccine of the present invention used to induce an immune response can be administered as a solution or suspension with pharmaceutically acceptable media.

Such pharmaceutically acceptable media can be, for example, water, phosphate-buffered saline, physiological saline or other physiologically buffered saline, or other solvents or media such as diol, glycerol, and oil (such as olive oil) or injectable organic ester. Pharmaceutically acceptable media may also contain liposomes or micelles and may contain immunostimulatory complexes prepared by mixing polypeptide or peptide antigens with detergents and glycosides (e.g., Quil A).

The immunogenic composition or vaccine of the present invention can be administered via a variety of routes to stimulate an immune response. For example, the immunogenic composition or vaccine can be delivered via subcutaneous, intradermal, intralymphatic, intramuscular, intratumoral, intravesical, intraperitoneal, intraperitoneal, and intracerebral routes.

Those skilled in the art will know how to select the appropriate route of delivery for the specific formulation of the inorganic salt-containing oil-in-water emulsion of the present invention.

In a preferred embodiment of the invention, a method of vaccination for the treatment or prevention of an infection in a mammal comprises using of the vaccine of the present invention, in which the vaccine is specifically administered intramuscularly. The vaccine may be administered as a single dose or preferably weekly or monthly for several times, such as twice, three times, or four times, depending on the primary immunization/booster immunization pattern. Appropriate dosage depends on various parameters.

In one aspect, the present application also relates to a use of the inorganic salt-containing oil-in-water emulsion of the present invention in the manufacture of a vaccine adjuvant, pharmaceutical composition, immunogenic composition, or vaccine composition, preferably, the vaccine comprising a protein vaccine, such as a varicella-zoster virus protein recombinant vaccine or a respiratory coronavirus protein recombinant vaccine.

In one aspect, the present application relates to a pharmaceutical formulation or composition, which comprises the inorganic salt-containing oil-in-water emulsion of the present invention.

A method for preparing a pharmaceutical formulation or composition comprises a step of combining the inorganic salt-containing oil-in-water emulsion of the present invention with a carrier and/or optionally one or more auxiliary components.

Generally, the formulation is prepared as follows: the inorganic salt-containing oil-in-water emulsion of the present invention is uniformly and tightly combined with a liquid carrier or a pulverized solid carrier, or both, and then the product is shaped if necessary.

The formulation or composition can be provided in single doses or in multi-doses in sealed containers (e.g., ampoules and vials), and can be stored under lyophilized conditions, requiring only the addition of a sterile liquid carrier, such as water for injection, just before use. Injectable solutions and suspensions can be prepared immediately from sterile powders, granules, and tablets of the types described above.

### Brief Description of the Drawings

Figure 1 exemplarily shows the appearance of the emulsion and aluminum particle-encapsulating emulsion prepared in Example 1 and the morphology thereof observed under electron microscope .
Figure 2 shows the effect of different processes used to prepare aluminum-encapsulating emulsions on efficacy in Example 6.
Figure 3 shows the antibody titers (A) and T-cell responses (B) induced by the aluminum-encapsulating emulsion in combination with different immunostimulants in Example 7.
Figure 4 shows the specific antibody binding titers and T-cell responses induced in mice by the aluminum-encapsulating emulsion and the aluminum-encapsulating emulsion combined with CpG1018 adjuvant, each formulated with the bivalent SARS-CoV-2 S protein (STFKA+B), in Example 8.
Figure 5 shows the specific antibody binding titers and T-cell responses induced in mice by the aluminum-encapsulating emulsion combined with CpG1018 adjuvant, formulated with respiratory syncytial virus (RSV) protein, in Example 9.
Figure 6 shows the specific antibody binding titers and T-cell responses induced in mice by the aluminum-encapsulating emulsion combined with CpG1018 adjuvant, formulated with varicella-zoster virus protein (VSV-gEgI), in Example 10.

### Specific Models for Carrying Out the present invention

The embodiments of the present invention are described in detail below with reference to examples. Unless otherwise specified, conditions in the examples were performed under conventional conditions or conditions recommended by the manufacturer. Reagents or instruments used, unless otherwise specified, were all commercially available conventional products.

### Preparation Example 1: Preparation of aluminum adjuvant

Aluminum chloride hexahydrate: purchased from Xilong Chemical
Disodium hydrogen phosphate dodecahydrate: purchased from Xilong Chemical
Sodium hydroxide: purchased from Xilong Chemical

Preparation of adjuvant: According to a phosphate/Al molar ratio of 0.15:1, 62 mM aluminum chloride solution was prepared by dissolving aluminum chloride in ultrapure water, obtaining a solution A; a solution containing 9.3 mM disodium hydrogen phosphate and 70 mM sodium hydroxide was prepared by dissolving disodium hydrogen phosphate and sodium hydroxide in ultrapure water, obtaining a solution B. The prepared solution B was added dropwise to solution A at a volume ratio of 1:1, thereby forming a suspension. The resulting suspension was sterilized by autoclaving at 121 °C for 60 minutes, and allowed to stand at room temperature for later use.

### Preparation Example 2: Preparation of aluminum hydroxide adjuvant

Aluminum chloride hexahydrate: purchased from Xilong Chemical
Sodium hydroxide: purchased from Xilong Chemical

Preparation of adjuvant: 3.6 mg/mL aluminum chloride solution was prepared, and defined as solution A; 0.04 mol/L sodium hydroxide solution was prepared, and defined as solution B. Following the preparation method of aluminum adjuvant in the literature (Li X, Aldayel AM, Cui Z. J Control Release. 2014;173:148-157), solution B was added dropwise to solution A at 10 rpm, then stirred at 500 rpm for 1 hour, thereby forming an aluminum hydroxide adjuvant. The aluminum hydroxide adjuvant was sterilized by autoclaving at 121 °C for 30 minutes, and allowed to stand at room temperature for later use.

### Preparation Example 3: Preparation of emulsion adjuvant

Squalene: Xi'an Tianzheng Pharmaceutical Excipients Co., Ltd.
Tween 80: Xi'an Tianzheng Pharmaceutical Excipients Co., Ltd.
Span 85: Xi'an Tianzheng Pharmaceutical Excipients Co., Ltd.

Preparation of adjuvant: 0.25 g of Tween 80 was suspended in 50 mL of 20 mM acetic acid-sodium acetate buffer solution (pH=6.0), which was defined as the aqueous phase; 0.25 g of Span 85 was mixed with 2.145 g of squalene, which was defined as the oil phase. The aqueous phase and the oil phase were mixed and homogenized using a benchtop homogenizer at 10,000 rpm for 2 minutes to form a crude emulsion, which was then homogenized three times using a high-pressure microfluidic nano-homogenizer to form an emulsion. Then, the emulsion was subjected to sterile filtration with a 0.22 µm filter and stored at 4 °C for later use.

### Preparation Example 4: Preparation of inorganic salt particle-encapsulating emulsion

Aluminum chloride, zinc chloride, calcium chloride, magnesium chloride, ferric chloride, manganese chloride: Xilong Chemical
Emulsion: from Preparation Example 3

Preparation of adjuvant: 37.5 mM inorganic metal salt was dissolved in 20 mM acetic acid-sodium acetate (pH=6.0), obtaining an inorganic metal salt solution. 3.125 mL of the inorganic metal salt solution was added dropwise to 28.5 mL of the emulsion at 100 µL/time (maintaining stirring at 500 to 1000 rpm during the addition). After the dropwise addition was complete, stirring was continued for 10 minutes, thereby forming an inorganic salt particle-encapsulating emulsion. The inorganic salt particle-encapsulating emulsion was subjected to sterile filtration with a 0.22 µm filter and stored at 4 °C for later use.

### Preparation Example 5: Preparation of aluminum-encapsulating emulsion (one-step method)

Aluminum chloride: Xilong Chemical
Squalene: Xi'an Tianzheng Pharmaceutical Excipients Co., Ltd.
Tween 80: Xi'an Tianzheng Pharmaceutical Excipients Co., Ltd.
Span 85: Xi'an Tianzheng Pharmaceutical Excipients Co., Ltd.

Preparation of adjuvant: 0.25 g of Tween 80 and 46.95 mg of aluminum salt were suspended in 50 mL of 20 mM acetic acid-sodium acetate buffer solution (pH=6.0), which was defined as the aqueous phase; 0.25 g of Span 85 was mixed with 2.145 g of squalene, which was defined as the oil phase. Alternatively, 0.25 g of Tween 80 was suspended in 50 mL of 20 mM acetic acid-sodium acetate buffer solution (pH=6.0), which was defined as the aqueous phase; 0.25 g of Span 85, 2.145 g of squalene, and 46.95 mg of aluminum salt were mixed, which was defined as the oil phase. The aqueous phase and the oil phase were mixed and subjected to high-speed shearing at 10,000 rpm for 2 minutes to form a crude emulsion, which was then homogenized three times using a high-pressure microfluidic nano-homogenizer to form an aluminum-encapsulating emulsion. Then, the aluminum-encapsulating emulsion was subjected to sterile filtration with a 0.22 µm filter. After the filtration, it was stored at 4 °C for later use.

### Preparation Example 6: Preparation of aluminum-encapsulating emulsion combined with immunostimulant

Aluminum-encapsulating emulsion: from Preparation Example 4
CpG1018: from Innovax
MPL: Sigma
PolyIC: Sigma
cGAMP: InvivoGen

Preparation of adjuvant: 10 µg/mL immunostimulant was dissolved in 20 mM acetic acid-sodium acetate, and added dropwise to an aluminum-encapsulating emulsion under sterile condition, mixed by inverting, and stored at 4 °C for later use.

### Example 1: Effects of different dispersion media on particle size and uniformity of aluminum hydroxide, emulsion, and aluminum-encapsulating emulsion

### (1) Sample preparation

The aluminum hydroxide adjuvant from Preparation Example 2 was diluted with 20 mM citric acid-sodium citrate, 20 mM acetic acid-sodium acetate, PBS, physiological saline, 20 mM Tris-HCl, 20 mM HEPES-HCl, 20 mM histidine-HCl, and pure water, respectively, to reach an aluminum content of 420 µg/mL. Then, the determination of particle size and PDI index was carried out directly, repeating three times.

The acetic acid-sodium acetate buffer solution from Preparation Example 3 was replaced with 20 mM citric acid-sodium citrate, PBS, physiological saline, 20 mM Tris-HCl, 20 mM HEPES-HCl, 20 mM histidine-HCl, and pure water, respectively, to prepare adjuvants. The obtained adjuvants were then diluted with the corresponding buffer solutions, and the determination of particle size and PDI index was carried out, repeating three times.

Following Preparation Example 4, aluminum-encapsulating emulsions were prepared using aluminum salts. The buffer solution conditions were acetic acid-sodium acetate, 20 mM Tris-HCl, 20 mM HEPES-HCl, and 20 mM histidine-HCl. Samples were retained before and after filtration using a 0.22 µm filter. The adjuvants before and after filtration were then diluted with the corresponding buffer solutions and their aluminum contents were determined.

Following Preparation Example 4, aluminum-encapsulating emulsions were prepared using aluminum salts. Six batches of aluminum-encapsulating emulsions were prepared using acetic acid-sodium acetate as the buffer condition. The freshly prepared adjuvants were visually observed and then diluted with the corresponding buffer solutions for electron microscopy. Subsequently, the adjuvants were stored at 4 °C. Samples were taken out at specified time intervals, diluted with the corresponding buffer solutions, and then determination of particle size and PDI index was carried out, repeating three times.

### (2) Appearance and morphology observation by electron microscopy

The emulsion and the aluminum particle-encapsulating emulsion was pipetted at a certain volume and placed into 4 mL ampoules. The layering was observed by photographing, and the appearance of the adjuvants was recorded. The adjuvants were diluted with 20 mM acetic acid-sodium acetate, and observed using a JEM-2100 transmission electron microscope (Electronics, Japan) or a Helios 5 UC scanning electron microscope (Thermo Fisher, USA).

### (3) Measurement of particle size and PDI:

The measurement was performed using a multi-angle particle size and high-sensitivity Zeta potential analyzer (Omni, Brookhaven) or a laser particle size analyzer (LS13320). The adjuvant was added to a sample cup, and the SOP was set for measurement.

### (4) Determination of metal content by ICP-OES:

a. Preparation of mixed metal element standard solutions: To a 50 mL centrifuge tube, standard solution of each metal element was accurately measured out and added, and 0.1 mol/L hydrochloric acid was added to make up to a volume of 20 mL to obtain a solution corresponding to the highest concentration point (STD5) of the standard curve. Then, the solution was serially diluted with hydrochloric acid (10 mL of hydrochloric acid was added to 10 mL of the previous standard working solution) to obtain STD4 to STD1 solutions sequentially. The final volume of each mixed standard curve solution should be 10 mL to ensure complete injection.
b. Preparation of test sample solution: A test sample was taken and equilibrated to room temperature, and mixed thoroughly with a vortex mixer. The test sample was accurately measured out, diluted to an appropriate factor with 0.1 mol/L hydrochloric acid solution, mixed well, and then allowed to stand for 1 hour to obtain a test sample solution. Preparation of blank control solution: 0.1 mol/L hydrochloric acid solution was taken and used as a blank control.
c. Determination: An argon gas cylinder was turned on (the gas pressure was adjusted to approximately 0.6 MPa); equilibration was carried out by blowing gas: ICP-OES instrument and software were turned on. In the software interface, "Purge Gas Flow" was selected from the "Dashboard", and the "Normal" mode was then chosen from the drop-down options. The instrument was equilibrated in this state for at least 1 hour. During equilibration, the software interface was observed, and "Torch compartment," "Plasma Gas Pressure," "Purge Gas Pressure," "Drain Flow," "Exhaust Flow," and "Optics Temperature" should all turn green. The water tank was turned on, and a wait of approximately 2 minutes was observed until the "Detector Water Flow" and "Detector Temperature" in the instrument status bar turned green. The sample inlet tube was then placed into ultrapure water, and the tube was correctly connected to the peristaltic pump. The "Pump Speed" in the interface was changed to 50, "Apply" was clicked, and it was observed whether liquid flowed out normally from the tube outlet. After rinsing for 1 to 2 minutes, the ignition procedure was carried out. Following ignition, "Dashboard" in the software interface was selected, the blue "Get Ready" icon at the top center of the operation interface was clicked, and "OK" was clicked in the pop-up window. An approximate wait of 2 to 5 minutes was required for successful ignition. Subsequently, the newly created template interface was accessed, and the element to be measured and its detection wavelength (e.g., Al, 396.152 nm) were checked in the right column "Analytes". The measurement was then commenced.
d. Result calculation

Four standard curves were plotted with the concentrations of the mixed standard solution as the horizontal coordinates and the corresponding peak areas as the vertical coordinates, and linear regression equations were established. The peak area of each element corresponding to the diluted test sample solution was substituted into the regression equations, multiplied by the dilution factor, and the metal element concentrations (ppm) of the test sample solution were calculated.

Experimental Results: As shown in Table 1, citric acid-sodium citrate and PBS buffer solutions increased the particle size of aluminum hydroxide to the micrometer level. In contrast, in conditions of physiological saline, acetic acid-sodium acetate, Tris-HCl, HEPES-HCl and histidine-HCl buffer solutions, there were no significant differences in the particle size of aluminum hydroxide. The effective diameters (Eff. Diam.) were all approximately 150 nm, with PDI values below 0.3. As shown in Table 2, in conditions of acetic acid-sodium acetate, physiological saline, Tris-HCl, HEPES-HCl, histidine-HCl, citric acid-sodium citrate, and PBS buffer solutions, there were no significant differences in the effective particle size of the emulsion adjuvant, all being around 200 nm, with PDI values below 0.25. Among these, the PDI values were the smallest in conditions of acetic acid-sodium acetate and citric acid-sodium citrate buffer solutions.

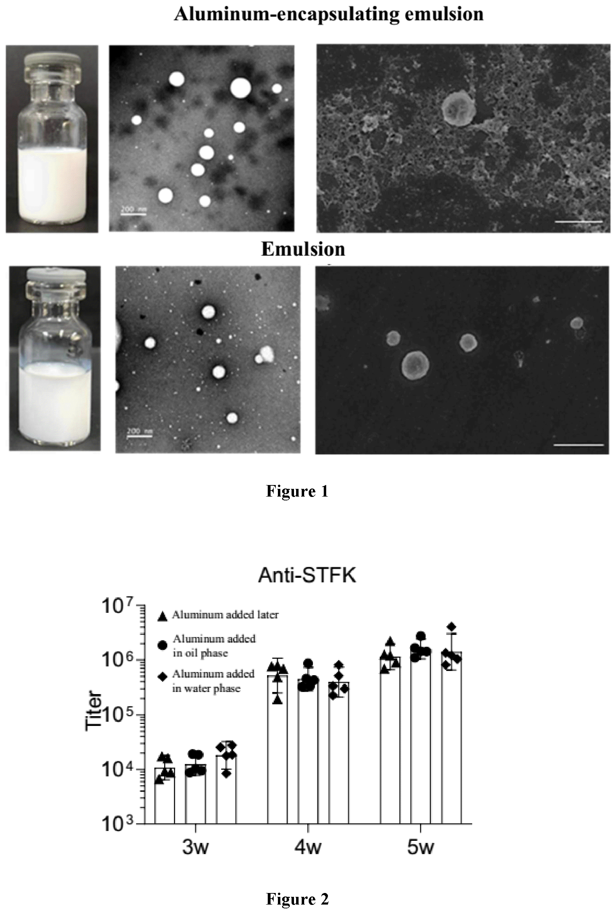

Considering the influence of the dispersion medium on aluminum hydroxide and the emulsion, Tris-HCl, HEPES-HCl, histidine-HCl, and acetic acid-sodium acetate buffer solutions were selected for the preparation of adjuvants for aluminum-encapsulating emulsions. As shown in Table 3, the loss of aluminum content before and after filtration was minimal under these four buffer solutions, all of which could be used as buffer solution conditions for aluminum-encapsulating emulsions. Subsequently, six batches of aluminum-encapsulating emulsions were prepared using an acetic acid-sodium acetate buffer solution and stored at 4 °C to observe changes in appearance, particle size, and uniformity. As shown in Figure 1, both the aluminum particle-encapsulating emulsion and the emulsion were white, opaque liquids. Under an electron microscope observation, the droplets of the aluminum particle-encapsulating emulsions appeared as spherical particles with aluminum salt particles distributed on the surface and outer side, while the emulsion only had smooth, spherical droplet structures. As shown in Table 4, the effective particle size of the six batches of aluminum-encapsulating emulsions fluctuated around 10 nm within four months, and the PDI indexes were less than 0.25, indicating good stability of the aluminum-encapsulating emulsions.

**Table 1. Effect of different dispersion media on particle size of aluminum hydroxide**

| Dispersion medium | Eff. Diam. (nm) | Diam. 10 (nm) | Diam. 50 (nm) | PDI index |
|---|---|---|---|---|
| Acetic acid-sodium acetate buffer solution | 151.5±5.316 | 81.68±3.607 | 151.5±5.316 | 0.262±0.009 |
| Physiological saline | 144.2±2.853 | 77.58±2.668 | 144.2±2.774 | 0.264±0.019 |
| Tris-HCl buffer solution | 145.8±4.544 | 78.80±3.628 | 145.8±4.544 | 0.260±0.015 |
| HEPES-HCl buffer solution | 146.8±1.679 | 79.47±2.071 | 146.8±1.679 | 0.258±0.016 |
| Histidine-HCl buffer solution | 143.6±4.113 | 76.04±1.652 | 143.6±4.113 | 0.279±0.014 |
| Citric acid-sodium citrate buffer solution | NA | 420.2±0.554 | 1414±6.293 | NA |
| PBS buffer solution | NA | 5268±23.59 | 9557±22.48 | NA |
| Pure water | 144.8±4.672 | 78.80±3.899 | 144.8±4.672 | 0.254±0.017 |

**Table 2. Emulsion particle size and uniformity in different conditions of dispersion media**

| Dispersion medium | Eff. Diam. (nm) | Diam. 10 (nm) | Diam. 50 (nm) | Diam. 90 (nm) | PDI index |
|---|---|---|---|---|---|
| Acetic acid-sodium acetate buffer solution | 193.42±0.73 | 65.74±1.26 | 96.71±0.37 | 142.31±3.03 | 0.095±0.010 |
| Physiological saline | 189.77±1.58 | 57.05±0.67 | 94.89±0.79 | 157.81±0.92 | 0.170±0.003 |
| Tris-HCl buffer solution | 192.94±1.05 | 55.79±0.38 | 96.47±0.53 | 166.81±0.72 | 0.200±0.001 |
| HEPES-HCl buffer solution | 190.91±1.15 | 58.12±1.26 | 95.46±0.58 | 156.81±1.60 | 0.162±0.011 |
| Histidine-HCl buffer solution | 186.41±1.15 | 55.39±1.08 | 93.20±0.57 | 156.88±2.00 | 0.179±0.011 |
| Citric acid-sodium citrate buffer solution | 193.42±0.73 | 65.74±1.26 | 96.71±0.37 | 142.31±3.03 | 0.095±0.010 |
| PBS buffer solution | 192.17±0.19 | 59.17±0.51 | 96.08±0.10 | 156.04±1.02 | 0.154±0.005 |
| Pure water | 213.38±2.50 | 59.09±0.15 | 106.69±1.25 | 192.66±4.94 | 0.237±0.013 |

**Table 3. Aluminum contents of aluminum-encapsulating emulsions before and after filtration in different conditions of dispersion media**

| Dispersion medium | Aluminum content before filtration (ppm) | Aluminum content after filtration (ppm) |
|---|---|---|
| Acetic acid-sodium acetate buffer solution | 90.20 | 88.70 |
| Tris-HCl buffer solution | 126.0 | 99.30 |
| HEPES-HCl buffer solution | 106.0 | 92.60 |
| Histidine-HCl buffer solution | 117.4 | 102.6 |

**Table 4. Changes in particle size and uniformity of six batches of aluminum-encapsulating emulsions after long-term storage**

| Aluminum-encapsulating emulsion | Eff. Diam. (nm) | Diam. 10 (nm) | Diam. 50 (nm) | PDI index |
|---|---|---|---|---|
| Storage for 1 month | | | | |
| lot1 | 188.45±2.44 | 127.36±11.5 | 188.45±2.44 | 0.104+0.038 |
| lot2 | 181.77±1.56 | 117.50+2.66 | 181.77±1.56 | 0.124+0.015 |
| lot3 | 186.67+0.88 | 129.58±10.6 | 186.67±0.88 | 0.091±0.042 |
| lot4 | 182.53±0.30 | 121.78±5.94 | 182.53±0.30 | 0.108±0.029 |
| lot5 | 184.08±1.08 | 134.14±11.4 | 184.08±1.08 | 0.069±0.029 |
| lot6 | 190.25±0.68 | 124.50±5.20 | 190.25±0.68 | 0.118±0.027 |

| Storage for 2 months | | | | |
|---|---|---|---|---|
| lot1 | 191.84±3.09 | 123.65±3.12 | 191.84±3.09 | 0.125±0.017 |
| lot2 | 185.27±4.44 | 122.00±3.74 | 185.27±4.44 | 0.112±0.004 |
| lot3 | 191.48±3.13 | 129.80+4.46 | 191.48±3.13 | 0.098+0.021 |
| lot4 | 188.84±0.46 | 116.51+4.89 | 188.84+0.46 | 0.154+0.028 |
| lot5 | 187.58 +2.03 | 120.49+0.44 | 187.58±2.03 | 0.127±0.005 |
| lot6 | 192.93 ±0.79 | 129.73±5.71 | 192.93 ±0.79 | 0.102±0.021 |

| Storage for 4 months | | | | |
|---|---|---|---|---|
| lot1 | 196.13 +2.49 | 121.46±1.23 | 196.13 ±2.49 | 0.151±0.015 |
| lot2 | 193.99±2.57 | 119.71±6.09 | 193.99±2.57 | 0.155±0.032 |
| lot3 | 197.47±0.52 | 118.92±3.52 | 197.47±0.52 | 0.171±0.021 |
| lot4 | 190.13±1.22 | 120.11±0.46 | 190.13±1.22 | 0.137±0.002 |
| lot5 | 196.02±2.08 | 119.47±4.91 | 196.02±2.08 | 0.162±0.022 |
| lot6 | 197.49±0.45 | 129.10±7.43 | 197.49±0.45 | 0.120±0.031 |

### Example 2: Particle size of emulsions encapsulated by other inorganic salt particles

According to Preparation Example 4, adjuvants were prepared using acetic acid-sodium acetate as buffer condition. The adjuvants were diluted at a certain factor with acetic acid-sodium acetate buffer solution, and measurement of particle size and PDI index was carried out, repeating three times. The method was the same as in Example 1.

Experimental results: As shown in Table 5, the effective particle size of the zinc-encapsulating, magnesium-encapsulating, calcium-encapsulating, iron-encapsulating, and manganese-encapsulating emulsion adjuvants was approximately 190 nm, and the PDI index was less than 0.15.

**Table 5. Particle size of other inorganic salt-encapsulating emulsions**

| Adjuvant name | Eff. Diam. (nm) | Diam. 10 (nm) | Diam. 50 (nm) | PDI index |
|---|---|---|---|---|
| Zinc-encapsulating emulsion | 180.4±3.879 | 116.6±2.411 | 180.4±3.879 | 0.123±0.001 |
| Magnesium-encapsulating emulsion | 173.1±1.541 | 109.0±2.375 | 173.1±1.541 | 0.140±0.008 |
| Calcium-encapsulating emulsion | 183.7±2.008 | 115.1±0.854 | 183.7±2.008 | 0.142±0.005 |
| Iron-encapsulating emulsion | 181.9±0.889 | 112.6±0.554 | 181.9±0.889 | 0.150±0.004 |
| Manganese-encapsulating emulsion | 190.1±2.389 | 124.8±8.948 | 190.1±2.389 | 0.118±0.035 |

### Example 3: Effect of squalene/surfactant ratio on particle size and uniformity of emulsions and aluminum-encapsulating emulsions

Following Preparation Example 3, the mass ratio of squalene/surfactant was adjusted to 2.6, 3.4, 4.3, 5.1, 6.9, and 8.6. The obtained emulsions were diluted with acetic acid-sodium acetate buffer solution at a certain factor, and measurement of particle size and PDI index was performed and repeated three times, using the method as described in Example 1. Here, the mass of the surfactant is the sum of the masses of Tween 80 and Span 85.

Emulsions with an effective particle size less than 220 nm were prepared into aluminum-encapsulating emulsions by adding aluminum salts according to the method of Preparation Example 4. After dilution with an acetic acid-sodium acetate buffer solution, the measurement of particle size and PDI index was performed and repeated three times, using the method as described in Example 1.

Experimental Results: As shown in Table 6, when the squalene/surfactant mass ratio was less than 5.1, the effective particle size of the emulsion was less than 220 nm, and the PDI was less than 0.2. Furthermore, the particle size of the prepared aluminum-encapsulating emulsion remained less than 220 nm, and the PDI was less than 0.2, indicating suitability for sterilization by filtration.

**Table 6. Effect of squalene/surfactant ratio on particle size and uniformity of emulsions and aluminum-encapsulating emulsions**

| Squalene/surfactant mass ratio | Emulsion | | Aluminum-encapsulating emulsion | |
|---|---|---|---|---|
| | Eff. Diam. (nm) | PDI index | Eff. Diam. (nm) | PDI index |
| 2.6 | 153.1±1.160 | 0.142±0.006 | 156.5±1.073 | 0.123±0.023 |
| 3.4 | 173.0±2.195 | 0.091±0.019 | 172.6±2.327 | 0.081±0.020 |
| 4.3 | 190.4±0.620 | 0.075±0.025 | 191.3±1.104 | 0.058±0.007 |
| 5.1 | 214.8±2.107 | 0.102±0.019 | 197.9±1.186 | 0.076±0.006 |
| 6.9 | 233.2±7.298 | 0.134±0.036 | NA | NA |
| 8.6 | 249.2±3.160 | 0.110±0.016 | NA | NA |

### Example 4: Effect of different aluminum concentrations on particle size and uniformity of aluminum-encapsulating emulsions

Following Preparation Example 4, the molar concentration of aluminum salt was adjusted to 18.75 mM, 75 mM, 112.5 mM, 225 mM, 337.5 mM, and 450 mM (i.e., aluminum content in the aluminum-encapsulating emulsions was 50 ppm, 200 ppm, 300 ppm, 600 ppm, 900 ppm, and 1200 ppm, respectively). Aluminum-encapsulating emulsions were diluted at a certain factor with acetic acid-sodium acetate buffer solution, and then measurement of particle size and PDI index was performed and repeated three times, using the method as described in Example 1.

Experimental results: As shown in Table 7, aluminum-encapsulating emulsions with an aluminum content greater than 1200 ppm resulted in particle sizes greater than 220 nm, while those with an aluminum content less than 1200 ppm resulted in particle sizes less than 220 nm. The PDI index was less than 0.2, indicating suitability for sterilization by filtration.

**Table 7. Effect of aluminum concentration on particle size and uniformity of aluminum-encapsulating emulsions**

| Aluminum content in aluminum-encapsulating emulsion (ppm) | Eff. Diam. (nm) | PDI index |
|---|---|---|
| 50 | 188.8±0.745 | 0.126+0.013 |
| 200 | 191.8±0.879 | 0.098±0.017 |
| 300 | 189.0±2.854 | 0.102+0.031 |
| 600 | 193.7±0.468 | 0.108±0.021 |
| 900 | 223.9±4.782 | 0.186±0.016 |
| 1200 | 295.8±54.22 | 0.191667 |

### Example 5: Effect of different surfactant concentrations on particle size of aluminum-encapsulating emulsions

Following Preparation Example 4, the mass of Span85 or Tween80 was adjusted to 0 g, 0.1 g, 0.15 g, and 0.3 g, respectively, (i.e., its concentration in the aluminum-encapsulating emulsion was 0% (w/v), 0.2% (w/v), 0.3% (w/v), and 0.6% (w/v), respectively). The aluminum-encapsulating emulsion was diluted at a certain factor with acetic acid-sodium acetate buffer solution, and then measurement of particle size and PDI index was performed and repeated three times, using the method as described in Example 1.

Experimental Results: As shown in Table 8, Span85 had little effect on the effective particle size of the aluminum-encapsulating emulsions. After adding different concentrations of Span85, the effective particle size of the aluminum-encapsulating emulsions was less than 220 nm, and the PDI index was below 0.20. As shown in Table 9, the Tween80 content significantly affected the effective particle size of the aluminum-encapsulating emulsions; when the concentration was less than 0.3% (w/v), the effective particle size of the aluminum-encapsulating emulsions was greater than 220 nm, and at some concentrations, the PDI index was greater than 0.20; when the concentration was greater than 0.3% (w/v), the effective particle size of the aluminum-encapsulating emulsions was less than 220 nm, and the PDI index was less than 0.20.

**Table 8. Effect of Span 85 concentration on particle size and uniformity of aluminum-encapsulating emulsions**

| Span 85 concentration | Eff. Diam. (nm) | PDI index |
|---|---|---|
| 0%(w/v) | 198.6±2.275 | 0.089±0.027 |
| 0.2%(w/v) | 193.0±1.134 | 0.094±0.014 |
| 0.3%(w/v) | 181.0±1.078 | 0.109±0.022 |
| 0.6%(w/v) | 184.9±2.146 | 0.105 ±0.011 |

**Table 9. Effect of Tween 80 concentration on particle size and uniformity of aluminum-encapsulating emulsions**

| Tween80 concentration | Eff. Diam. (nm) | PDI index |
|---|---|---|
| 0%(w/v) | 1249+469.7 | 0.312+0.047 |
| 0.2%(w/v) | 259.6±2.441 | 0.082±0.034 |
| 0.3%(w/v) | 219.9±2.729 | 0.053±0.008 |
| 0.6%(w/v) | 175.6± 1.708 | 0.109+0.016 |

### Example 6: Effects of different preparation processes on particle size, uniformity, and efficacy of aluminum-encapsulating emulsions

Following Preparation Example 4, aluminum-encapsulating emulsion was prepared by adding aluminum salt (i.e., aluminum was added later). Following Preparation Example 5, aluminum-encapsulating emulsion was prepared using a one-step method. After dilution with acetic acid-sodium acetate buffer solution at a certain factor, measurement of particle size and PDI index was performed and repeated three times, using method as described in Example 1. The aluminum-encapsulating emulsions prepared according to Preparation Example 4 and Preparation Example 5 were combined with the COVID-19 antigen (1 µg/mouse, STFK, see Cell Host & Microbe 2022, 30, 1732 to 1744) at a volume ratio of 9.37:1.00 to form vaccines, respectively. The antigen was diluted with 20 mM acetic acid-sodium acetate buffer solution. The antigen adsorption was then carried out by incubation at 4 °C overnight, and then intramuscular injection into C57BL/6 mice was carried out, and the titers of generated specific antibody were detected. The specific method was as follows:
Experimental animals: C57BL/6 mice (purchased from Shanghai SLAC Laboratory Animal Co., Ltd.), aged 6 to 7 weeks, 5 mice per group, female.

Experimental groups: (1) aluminum added later; (2) aluminum in oil phase; and (3) aluminum in aqueous phase.

Immunization protocol: 1 µg antigen/mouse, 150 µL per mouse. Two immunizations were administered at week 0 and week 3. Blood was collected from orbital rim at week 3, week 4, and week 5 for serum antibody titer determination.

Enzyme-linked immunosorbent assay (ELISA) was used to determine antibody binding titers, and the specific method was as follows:
Plate coating and blocking: Recombinant S protein STFK was diluted to 1 µg/mL with CB9.6 and added to a 96-well ELISA plate at 100 µL/well. The plate was incubated at 37°C for 2 hours, then washed once with PBST buffer solution (i.e., PBS containing 0.05% Tween-20). 200 µL of blocking buffer solution was added to each well, and the plate was incubated at 37°C for another 2 hours for blocking. Excess liquid was removed, and the plate was spin-dried for later use.

Serum dilution: 100 µL of serum diluted 100 or 300 times was added to each well, and the plate was incubated at 37°C for 1 hour.

Addition of enzyme-labeled antibody: After the plate was washed 5 times with PBST buffer solution, 100 µL of HRP-conjugated goat anti-mouse IgG (H+L) (BEYOTIME, A0201) diluted at a certain factor was added, and the plate was incubated at 37°C for 0.5 hours.

Colorimetric development: The enzyme-labeled antibody in the wells was discarded, the plate was washed 5 times with PBST, and spin-dried. Equal volumes of colorimetric reagents A and B were mixed added at 100 µL/well, and incubated at 37°C for 10 minutes.

Termination: 2 M sulfuric acid termination solution was added at 50 µL/well to terminate the reaction.

Plate reading: The dual wavelengths for detection were set to 450 nm and 630 nm on a microplate reader, and the OD values of each reaction well were measured.

Experimental results: As shown in Table 10 and Figure 2, there were no significant differences in particle size, PDI index, and binding titer of aluminum-encapsulating emulsions prepared by different processes. This indicated that aluminum salt particles could be added after emulsification or during the process of forming emulsions, and could be added in the aqueous or oil phase.

**Table 10. Effects of different preparation processes on particle size and uniformity of aluminum-encapsulating emulsions**

| Different processes | Eff. Diam. (nm) | PDI index |
|---|---|---|
| Aluminum added later (according to Preparation Example 4) | 188.1±1.431 | 0.090±0.017 |
| Aluminum in oil phase (according to Preparation Example 5) | 187.8±2.163 | 0.105±0.040 |
| Aluminum in aqueous phase (according to Preparation Example 5) | 185.8±1.730 | 0.128±0.018 |

### Example 7: Specific antibody binding titers and T-cell responses in mice produced by aluminum-encapsulating emulsions after they were combined with different immunostimulants and formulated with bivalent SARS-CoV-2 S protein (STFKA+B)

Immunostimulants were added to the aluminum-encapsulating emulsion prepared according to the method of Preparation Example 6, and then formulated with SARS-CoV-2 S protein (STFKA+B, see, Cell Host & Microbe 2022, 30. (1732 to 1744) by mixing at a volume ratio of 9.37:1.00 to form vaccines, and the antigens were diluted with 20 mM acetic acid-sodium acetate. The aluminum adjuvant (Preparation Example 1) was mixed with STFKA+B at a volume ratio of 1:1 to form a vaccine, and physiological saline was added for antigen dilution. Incubation was performed at 4 °C overnight for antigen adsorption, followed by intramuscular injection into C57BL/6 mice, and the titers of generated specific antibody were measured. The specific method was as follows:
Experimental animals: C57BL/6 mice (purchased from Shanghai SLAC Laboratory Animal Co., Ltd.), aged 6 to 7 weeks, 6 mice per group, female.

Experimental groups: (1) aluminum adjuvant; (2) aluminum particle-encapsulating emulsion (ALE); (3) aluminum particle-encapsulating emulsion combined with CpG1018 (ALE+CpG); (4) aluminum particle-encapsulating emulsion combined with PolyIC (ALE+PolyIC); (5) aluminum particle-encapsulating emulsion combined with cGAMP (ALE+cGAMP); and (6) aluminum particle-encapsulating emulsion combined with MPL (ALE+MPL).

Immunization protocol: The antigens were administered at 1 µg/animal, 150 µL per animal. Two immunizations were administered at week 0 and week 3. At week 5, blood was collected from orbital rim for determination of antibody titer in serum and T-cell response.

Antibody binding titer was determined using enzyme-linked immunosorbent assay (ELISA), the procedure of which can be found in Example 6.

T-cell response was determined using the ELISPOT method, and the specific method was as follows:
Single-cell suspension was obtained by gently squeezing spleen tissue through a cell sieve with PBS + 2% (v/v) fetal bovine serum (GIBCO). Red blood cells were then lysed with erythrocyte lysis buffer solution (Solarbio) and washed twice. The cell suspension was seeded at 5×10⁵ cells per well in a 96-well plate, and stimulated at 37 °C for 12 h with a mixture of Spike peptides (0.66 µg/mL, DOI: 10.1126/scitranslmed.abg114). IFN-γ ELISPOT assay was then performed using IFN-γ ELISPOT kit (Dakewe Biotech) according to the manufacturer's instructions. Spots were counted using an ImmunSpot@S5 UV analyzer. The number of IFN-γ positive cells was calculated as: number of spots in stimulated wells - number of spots in unstimulated wells.

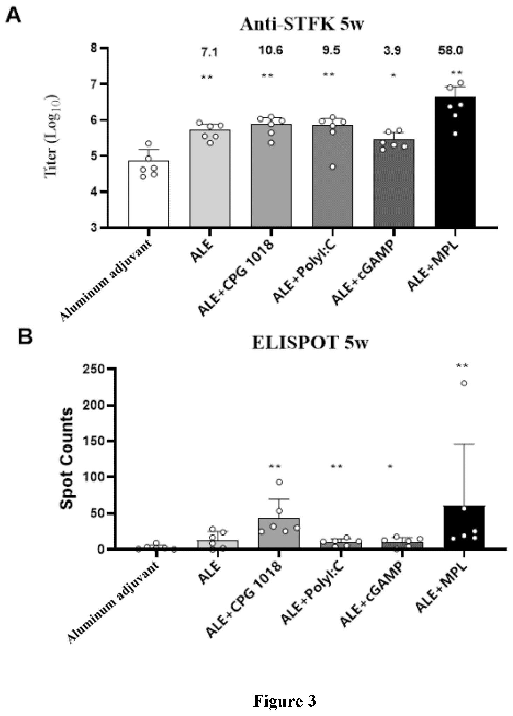

Experimental results: As shown in Figure 3, the aluminum-encapsulating emulsions could induce an antibody titer 7 times higher than that of the aluminum adjuvant. Combined with immunostimulatory small molecules, it still could induce higher antibody titers (3-60 times higher) and higher levels of T-cell responses than the aluminum adjuvant.

### Example 8: Specific antibody binding titers and T-cell responses in mice produced by aluminum-encapsulating emulsion and aluminum-encapsulating emulsions combined with CpG1018 adjuvant, after formulated with bivalent COVID-19 S protein (STFKA+B)

Vaccine preparation was the same as in Example 7. The prepared vaccines were incubated at 4 °C overnight for antigen adsorption, and then injected intramuscularly into C57BL/6 mice. The specific antibody titers and T-cell responses were measured. The specific method was as follows:
Experimental animals: C57BL/6 mice (purchased from Shanghai SLAC Laboratory Animal Co., Ltd.), aged 6 to 7 weeks, 6 mice per group, female.

Experimental groups: (1) aluminum adjuvant; (2) Addavax (purchased from InvivoGen); (3) aluminum particle-encapsulating emulsion (ALE); (4) aluminum adjuvant + CpG1018; (5) Addavax + CpG1018; and (6) ALE + CpG1018.

Immunization protocol: The antigens were administered at 1 µg/mouse, 150 µL per mouse. Two immunizations were administered at week 0 and week 3. At week 5, blood was collected from orbital rim for the determination of serum antibody titers and T-cell responses.

Antibody detection and T-cell response detection were performed as in Example 7.

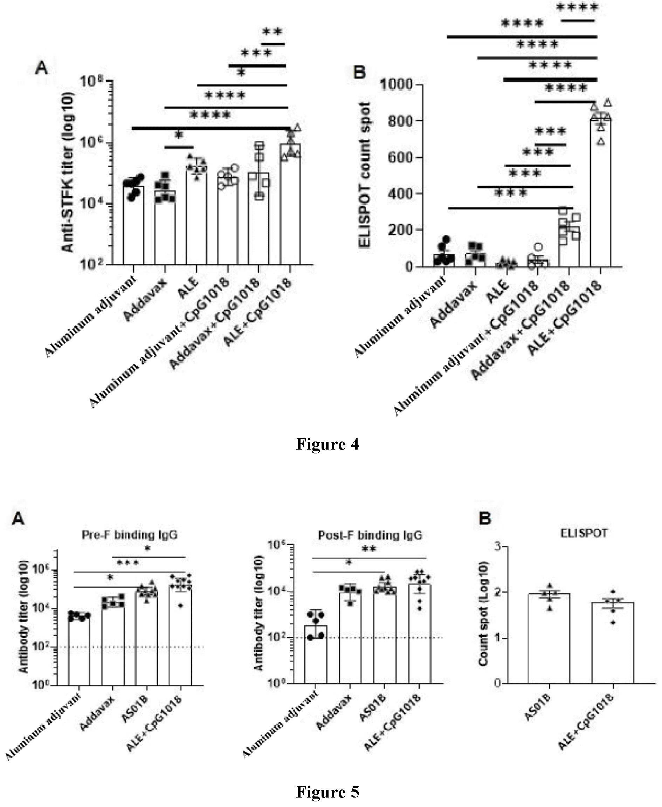

Experimental results: As shown in Figure 4, the aluminum-encapsulating emulsion combined with CpG1018 could induce an antibody titer that was 30, 38, 7, 3, and 5 times higher than those induced by aluminum adjuvant, Addavax, ALE, aluminum adjuvant + CpG1018, and Addavax + CpG1018, respectively. The aluminum-encapsulating emulsion combined with CpG1018 could induce T-cell response that was 11, 11, 34, 5, and 4 times higher than those induced by aluminum adjuvant, Addavax, ALE, aluminum adjuvant + CpG1018, and Addavax + CpG1018, respectively.

### Example 9: Specific antibody binding titers and T-cell responses in mice produced by aluminum-encapsulating emulsion combined with CpG1018 adjuvant and formulated with respiratory syncytial virus (RSV)

Vaccine preparation was the same as in Example 7. RSV antigen was obtained from Xiamen Innovax. The prepared vaccines were incubated at 4 °C overnight for antigen absorption, and then injected intramuscularly into Balb/C mice. The specific antibody binding titers and T-cell responses were measured. The specific method was as follows:
Experimental animals: Balb/C (purchased from Shanghai SLAC Laboratory Animal Co., Ltd.), aged 6 to 7 weeks, 5 mice per group, female.

Experimental groups: (1) aluminum adjuvant; (2) Addavax (purchased from InvivoGen); (3) AS01B (commercially purchased; this adjuvant could induce strong humoral and cellular immune responses in current human vaccines and had been used in Shingrix shingles vaccine); and (4) ALE+CpG1018.

Immunization protocol: The antigens were administered at 1 µg/mouse, 150 µL per mouse. Immunizations were performed at week 0 and week 2. Blood was collected from orbital rim at week 5 for serum antibody titer determination, and T-cell response was determined at week 6.

Antibody detection and T-cell response detection were the same as in Example 7.

Experimental results: As shown in Figure 5, the aluminum particle-encapsulating emulsion combined with CpG1018 (i.e., ALE+CpG1018) could induce antibody titer and T-cell response that were stronger than those induced by the aluminum adjuvant and the commercial emulsion (i.e., Addavax), and comparable to those induced by AS01B adjuvant.

Example 10: Specific antibody binding titers and T-cell responses in mice produced by aluminum-encapsulating emulsion combined with CpG1018 adjuvant and formulated with varicella-zoster virus protein (VZV-gEgI).

The vaccine preparation was the same as in Example 7. VZV-gEgI antigen was obtained from Xiamen Innovax. The prepared vaccines were incubated at 4 °C overnight for antigen absorption, and then injected intramuscularly into Balb/C mice. The specific antibody binding titers and T-cell responses were measured. The specific method was as follows:
Experimental animals: C57BL/6 (purchased from Shanghai SLAC Laboratory Animal Co., Ltd.), aged 6 to 7 weeks, 6 mice per group, female.

Experimental groups: (1) aluminum adjuvant; (2) ALE + CpG1018; and (3) commercial Shingrix (from Xiamen Innovax).

Immunization protocol: Groups 1 and 2 received the antigen at 5 µg/mouse, 150 µL per mouse; Group 3 received the commercial vaccine (containing 50 µg of antigen, 0.5 mL per dose), and the vaccine was administered at 50 µL per mouse (i.e., containing 5 µg of antigen). Immunizations were performed at week 0 and week 4. At week 5, blood was collected from orbital rim for determination of antibody titer in serum. At week 6, T-cell response was determined.

Antibody detection and T-cell response detection were performed as in Example 7.

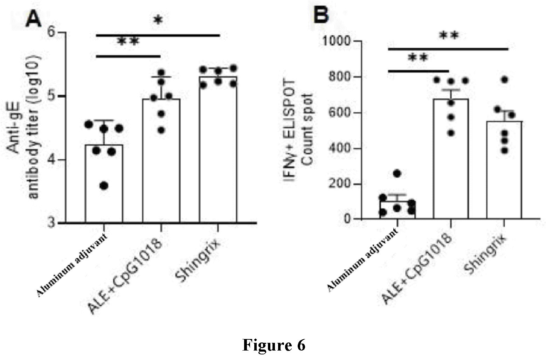

Experimental results: As shown in Figure 6, the aluminum particle-encapsulating emulsion combined with CpG1018 (i.e., ALE + CpG1018) could induce antibody titer and T-cell response stronger than those of the aluminum adjuvant, and comparable to those of the commercial varicella-zoster vaccine (Shingrix).

The above experiments demonstrate that the inorganic salt particle-encapsulating emulsion of the present invention can improve antibody levels and T-cell responses, resulting in a more comprehensive, significant, and durable immune protection effect. The inorganic salt particle-encapsulating emulsion of the present invention has a particle size of less than 220 nm, allowing for sterilization by filtration to ensure the sterility of adjuvant. This solves the problem of adjuvants containing inorganic salts and squalene being unable to undergo hydrothermal sterilization, which is beneficial for large-scale industrial production. Furthermore, compared to commercially available adjuvants containing QS-21 (which is a triterpenoid glycoside purified from the bark extract of *Quillaja saponaria Molina*)*,* such as AS01B, the adjuvant of the present invention has a wider and more readily available source of raw materials, which can reduce vaccine costs and facilitate vaccine production and promotion.

The foregoing descriptions are merely preferred embodiments of the present invention and are not intended to limit the present invention. Any modification, equivalent replacement, or improvement made within the spirit and principle of the present invention shall be included within the protection scope of the present invention.

## Claims

1. An inorganic salt-containing oil-in-water emulsion, wherein the inorganic salt is one or more selected from the group consisting of aluminum salt, zinc salt, calcium salt, magnesium salt, iron salt, and manganese salt; the emulsion comprises an aqueous phase selected from the group consisting of acetic acid-sodium acetate buffer solution, physiological saline, Tris-HCl buffer solution, HEPES-HCl buffer solution, and histidine-HCl buffer solution; the emulsion comprises an oil phase which is one or more selected from the group consisting of squalene and squalane; the emulsion further comprises a surfactant, and has an oil phase/surfactant mass ratio of less than 5.1; the inorganic salt and the surfactant are distributed at the interface between the oil phase and the aqueous phase; the effective particle size of the inorganic salt-containing emulsion is less than 220 nm.

2. The inorganic salt-containing oil-in-water emulsion according to claim 1, wherein the inorganic salt is a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate.

3. The inorganic salt-containing oil-in-water emulsion according to claim 1 or 2, wherein the emulsion has a content of inorganic metal element is less than 1200 ppm, for example, 50 to 1200 ppm.

4. The inorganic salt-containing oil-in-water emulsion according to any one of claims 1 to 3, wherein the surfactant is selected from the group consisting of Tween (e.g., Tween 80), Span (e.g., Span 85), Triton X-100, or combinations thereof.

5. The inorganic salt-containing oil-in-water emulsion according to any one of claims 1 to 4, wherein the oil phase/surfactant mass ratio is less than 5.1.

6. The inorganic salt-containing oil-in-water emulsion according to any one of claims 1 to 5, wherein the surfactant comprises Tween 80, and the Tween 80 has a concentration of greater than 0.3% (w/v).

7. The inorganic salt-containing oil-in-water emulsion according to any one of claims 1 to 6, further comprising an additional immunostimulant or peptide, such as CpG1018, MPL, PolyIC, or cGAMP.

8. The inorganic salt-containing oil-in-water emulsion according to any one of claims 1 to 7, wherein the emulsion has a change in particle size of not more than 10 nm and the emulsion does not separate into layers after being stored at 4 °C for at least 4 months.

9. A method for preparing an inorganic salt-containing oil-in-water emulsion, the method comprises the following steps:
S1: preparing an oil-in-water emulsion using an acetic acid-sodium acetate buffer solution, physiological saline, Tris-HCl buffer solution, HEPES-HCl buffer solution, or histidine-HCl buffer solution as an aqueous phase, squalene and/or squalane as an oil phase, and a surfactant; wherein the emulsion has an oil phase/surfactant mass ratio of less than 5.1; the emulsion has an effective particle size of less than 220 nm, and a PDI of 0.07 to 0.2;
S2: adding an inorganic salt solution dropwise to the emulsion prepared in S1; wherein the inorganic salt is one or more selected from the group consisting of aluminum salt, zinc salt, calcium salt, magnesium salt, iron salt, and manganese salt; the solvent of the inorganic salt solution is the same as the aqueous phase in S1; the inorganic salt in the inorganic salt solution has a concentration of less than 450 mM; continuously stirring after the addition is complete, and then sterilizing by filtration through a 0.22 µm filter.

10. The method according to claim 9, comprising one or more of the following features:
(1) the inorganic salt is a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate;
(2) the buffer solution used in S1 or S2 has a concentration of 20 to 100 mM;
(3) the inorganic salt solution has a concentration of 18.75 mM to 450 mM.

11. The method according to claim 9 or 10, wherein S1 comprises: suspending surfactant 1 in a certain volume of buffer solution or physiological saline, and defining it as solution A; suspending surfactant 2 in squalene and/or squalane, and defining it as solution B; adding solution A to solution B, performing high-speed shearing to form a crude emulsion, and then homogenizing for several times to form emulsion 1;
preferably, surfactant 1 (e.g., Tween 80) in emulsion 1 has a content of 0.1% to 0.5% (w/v);
preferably, surfactant 2 (e.g., Span 85) in emulsion 1 has a content of 0.1% to 0.5% (w/v);
preferably, squalene and/or squalane in emulsion 1 has a volume content of 1% to 5% (v/v).

12. The method according to any one of claims 9 to 11, wherein S2 comprises: dissolving the inorganic salt in a buffer solution or physiological saline to obtain an inorganic salt solution; adding the inorganic salt solution dropwise to the emulsion obtained in S1 at a volume ratio of 1:9 to 1:10 at a rate of 100 to 500 µL/time, continuously stirring after the addition is complete, and then sterilizing by filtration through a 0.22 µm filter.

13. A method for preparing an inorganic salt-containing oil-in-water emulsion, the method comprises the following steps:
S1': suspending a first surfactant and an inorganic salt in an acetic acid-sodium acetate buffer solution, physiological saline, Tris-HCl buffer solution, HEPES-HCl buffer solution, or histidine-HCl buffer solution, and defining it as aqueous phase; mixing a second surfactant with squalene and/or squalane, and defining it as oil phase; or,
suspending a first surfactant in an acetic acid-sodium acetate buffer solution, physiological saline, Tris-HCl buffer solution, HEPES-HCl buffer solution, or histidine-HCl buffer solution, and defining it as aqueous phase; mixing a second surfactant, as well as squalene and/or squalane with an inorganic salt, and defining it as oil phase; the inorganic salt is one or more selected from the group consisting of aluminum salt, zinc salt, calcium salt, magnesium salt, iron salt, and manganese salt;
S2': mixing the aqueous phase and the oil phase, performing high-speed shearing to form a crude emulsion, then homogenizing for several times to form an inorganic salt-encapsulatingemulsion, and sterilizing by filtration using a 0.22 µm filter.

14. The method according to claim 13, comprising one or more of the following features:
(1) the inorganic salt is a halide or sulfate, such as aluminum chloride, zinc chloride, calcium chloride, magnesium chloride, ferric chloride, ferrous chloride, manganese chloride, aluminum sulfate, zinc sulfate, calcium sulfate, magnesium sulfate, ferric sulfate, ferrous sulfate, or manganese sulfate;
(2) in S1', the inorganic salt in the aqueous phase has content of 1.92 to 46.2 mM;
(3) in S1', the inorganic salt in the oil phase has a content of 35.2 to 845 mM;
(4) in S1', the first surfactant in the inorganic salt-encapsulating emulsion has a content of 0.1% to 0.5% (w/v);
(5) in S1', the second surfactant in the inorganic salt-encapsulating emulsion has a content of 0.1% to 0.5% (w/v); and
(6) the first surfactant and the second surfactant are Tween 80 and Span 85, respectively.

15. A vaccine adjuvant, pharmaceutical composition, or immunogenic composition, which comprises the inorganic salt-containing oil-in-water emulsion according to any one of claims 1 to 8 or an inorganic salt-containing oil-in-water emulsion prepared by the method according to any one of claims 9 to 14.

16. A vaccine composition, which comprises an antigen and the inorganic salt-containing oil-in-water emulsion according to any one of claims 1 to 8 or an inorganic salt-containing oil-in-water emulsion prepared by the method according to any one of claims 9 to 14.

17. Use of the inorganic salt-containing oil-in-water emulsion according to any one of claims 1 to 8 or an inorganic salt-containing oil-in-water emulsion prepared by the method according to any one of claims 9 to 14 in manufacture of a vaccine adjuvant, pharmaceutical composition, drug delivery carrier, immunogenic composition, or vaccine composition.
